# EUROPÄISCHE PATENTSCHRIFT

(11) **EP 1 981 570 B1**
(45) Veröffentlichungstag und Bekanntmachung des Hinweises auf die Patenterteilung: **19.10.2016**
(21) Anmeldenummer: 07700117.0
(22) Anmeldetag: 15.01.2007
(51) Int. Cl.: A61M 5/315, A61M 5/24, A61M 5/31, A61M 5/50

(54) **VORSCHUBSTANGE MIT KOPPELELEMENT**
PUSHER WITH A COUPLING ELEMENT
POUSSOIR POURVU D'UN ÉLÉMENT DE COUPLAGE

(30) Priorität: 17.01.2006 DE 102006002383; 01.02.2006 DE 102006004562
(43) Veröffentlichungstag der Anmeldung: 22.10.2008
(73) Patentinhaber: TecPharma Licensing AG, 3400 Burgdorf (CH)
(72) Erfinder: MOSER, Ulrich, 3412 Heimiswil (CH); SCHRUL, Christian, 3400 Burgdorf (CH); HIRSCHEL, Jürg, 5000 Aarau (CH)
(86) Internationale Anmeldenummer: PCT/CH2007/000019
(87) Internationale Veröffentlichungsnummer: WO 2007/082401

(56) Entgegenhaltungen:
- EP-A1- 0 373 321
- WO-A-00/62839
- WO-A1-95/04563
- WO-A1-2004/007000
- DE-C1- 19 730 999
- DE-C1- 19 900 792
- US-A- 5 807 346
- US-A1- 2001 009 990
- US-A1- 2004 186 441
- US-A1- 2005 222 540

## Beschreibung

Die vorliegende Erfindung bezieht sich auf eine Vorrichtung zur Abgabe einer Substanz, insbesondere eine Injektionsvorrichtung oder einen Pen, der für die dosierte Verabreichung eines injizierbaren Produktes ein Dosierelement aufweist. Das Injektionsgerät dient vorzugsweise der Selbstverabreichung des Produkts, insbesondere als Einweginjektor, welcher zum Beispiel mit einer einfachen oder Zweikammerampulle verwendet werden kann.

Injektionspens sind aufgrund ihrer leicht handhabbaren Form zur Selbsverabreichung von Medikamten weit verbreitet. Insbesondere bei der Selbsverabreichung eines Medikaments, wie beispielsweise Insulin oder eines Hormons, kommt der einfachen Handhabbarkeit, der Exaktheit der Dosierung und der Sicherheit bei der Einstellung der Dosierung große Bedeutung zu.

Aus der DE 10 2004 004 310 der Anmelderin ist ein Injektionsgerät bekannt, das ein Dosierglied aufweist, welches relativ zu dem Gehäuse des Injektionsgerätes eine Vortriebsbewegung in eine Vortriebsrichtung und eine Dosierbewegung gegen die Vortriebsrichtung ausführt und mit einer Fördereinrichtung so gekoppelt ist, dass die Vortriebsbewegung eine Förderbewegung der Fördereinrichtung bewirkt, wobei die Länge einer die auszuschüttende Dosis bestimmenden Wegstrecke, die das Dosierglied bei der Vortriebsbewegung zurücklegt, durch die Dosierbewegung eingestellt wird. Eine erste Rasteinrichtung wird durch das Gehäuse in einem das Dosierglied umgebenen Hülsenabschnitt gebildet und eine zweite Rasteinrichtung wird von dem Dosierglied oder einem separaten, zusätzlichen Dosierrastkörper gebildet, wobei ein Rasteingriff der Rasteinrichtungen durch eine drehbewegungsfreie Dosierbewegung des Dosiergliedes oder des sparaten, zusätzlichen Dosierrastkörpers lösbar ist.

Aus der US 5,807,346 ist ein Messinstrument zum Abgeben verschiedener Dosen einer Flüssigkeit bekannt, welches ein Reservoir zur Aufnahme der Flüssigkeit, ein innerhalb und axial angeordnetes bewegbares Zahnstangenelement und ein Schubelement aufweist, welches von dem Benutzer betätigbar ist. Auf dem Zahnstangenelement sind ringförmige Einkerbungen vorgesehen, wobei das Gehäuse verschiedene axiale Schlitze aufweist, welche über dessen Peripherie verteilt sind. An dem Schubelement ist ein nach außen vorstehender Stift vorgesehen, welcher in einen der axialen Schlitze des Gehäuses eingreifen kann, wobei das Einstellelement vom Benutzer einfach herausgezogen werden kann.

Aus der EP 0 713 403 B1 ist eine Spritze mit einer Trommel bekannt, die einen Zylinder definiert oder aufweist, wobei der Zylinder eine Düse an einem Ende aufweist und einen Kolben einschließt, der in Längsrichtung innerhalb des Zylinders gleitbar ist, wobei der Abstand, um den sich der Kolben innerhalb des Zylinders bewegen kann, das Volumen definiert, dass durch die Spritze verabreicht wird, wobei der Kolben treibend mit einem Schieber gekoppelt ist, der derart angeordnet ist, dass er sich parallel zu dem Kolben bewegt, wobei der Längsabstand, um den der Kolben innerhalb des Zylinders gleiten kann, durch die Bewegungsbegrenzungen einer Anschlagfläche an der Trommel oder dem Schieber bezüglich Endanschlägen an dem Schieber bzw. der Trommel definiert ist. Die Spritze ist in einem Zustand verriegelbar, in dem sich die Anschlagfläche und die Endanschläge bezüglich einanander nur innerhalb von Bewegungsbegrenzungen bewegen können, die aus zwei oder mehr wählbaren vorbestimmten Bewegungsbegrenzungen gewählt werden, und dadurch, dass die Anschlagfläche einen Einsatz aufweist, der an eine Öffnung in der Trommel oder dem Schieber derart einführbar ist, dass er von diesen vorsteht und von diesen nur mit Schwierigkeiten oder durch Verwendung eines speziellen Werkzeugs entfernbar ist, wobei der vorstehende Teil des Einsatzes eine Anschlagfläche bildet, und wobei die Stelle einer jeden derartigen Öffnung bezüglich der Endanschläge einen der vorgewählten Bewegungsabstände definiert.

Es ist eine Aufgabe der vorliegenden Erfindung eine Injektionsvorrichtung vorzuschlagen, welche die einfache Einstellbarkeit verbessert bei gleichzeitiger Verringerung der Wahrscheinlichkeit von Fehlbedienungsvorgängen.

Diese Aufgabe wird durch eine Injektionsvorrichtung gemäß Patentanspruch 1 gelöst. Vorteilhafte Ausführungsformen ergeben sich aus den abhängigen Patentansprüchen.

Gemäß einem ersten Aspekt weist eine erfindungsgemäße Injektionsvorrichtung eine Aufnahmevorrichtung für eine abzugebende Substanz auf, in welcher die abzugebende Substanz entweder unmittelbar eingebracht werden kann oder in einem Behältnis, zum Beispiel in einer Ampulle, eingelegt werden kann, so dass die Aufnahmevorrichtung zum Beispiel als Ampullenhalter ausgebildet ist. Diese Aufnahmevorrichtung ist relativ zur Injektionsvorrichtung bewegbar, wobei zum Beispiel ein die Aufnahme oder den Ampullenhalter bildendes Element der Injektionsvorrichtung in diese oder in einen Teil davon, zum Beispiel in das Gehäuse der Injektionsvorrichtung einschiebbar oder einschraubbar ist, so dass zum Beispiel bei Verwendung einer bekannten Zweikammerampulle durch den Einschub- oder Einschraubvorgang die in der Zweikammerampulle enthaltenen Substanzen abgemischt und somit für die Abgabe an einen Patienten vorbereitet werden können. Weiterhin ist ein Dosier- oder Einstellelement, wie zum Beispiel ein Drehknopf, zum Einstellen der aus der Injektionsvorrichtung abzugebenden Dosis der Substanz vorgesehen, wobei die Menge oder Dosis der abzugebenden Substanz zum Beispiel durch eine Drehposition des Einstell- oder Dosierelements gewählt oder festgelegt werden kann. Das Dosierelement ist in einem Ausgangszustand in die erfindungsgemäße Injektionsvorrichtung vorzugsweise soweit eingeschoben, dass es von einem Benutzer nicht oder nur sehr schwer gegriffen und bevorzugt auch nicht einfach herausgezogen werden kann. Erfindungsgemäß ist die in die Injektionsvorrichtung einführbare oder einschiebbare Aufnahmevorrichtung für die abzugebende Substanz, wie zum Beispiel ein einschraub- oder einschiebbarer Ampullenhalter, so mit dem Einstellelement gekoppelt, dass während des Einschiebens oder nach dem vollständigen oder fast vollständigen Einschieben der Substanzaufnahmevorrichtung in die Injektionsvorrichtung oder in ein Gehäuse, das Einstellelement aus der Injektionsvorrichtung oder einem Gehäuse davon zumindest zum Teil so herausgeschoben wird, dass es von einem Benutzer zum Beispiel zum Einstellen einer Dosis betätigt werden kann. Das Dosier- oder Einstellelement kann zum Beispiel als ein an sich bekannter drehbarer Dosierknopf ausgebildet sein, welcher sich am proximalen Ende der Injektionsvorrichtung befindet und beispielsweise fest mit einer Dosierknopfhülse verbunden ist, die axial innerhalb der Injektionsvorrichtung verschiebbar und bevorzugt aus dieser ausschiebbar ist, um den Dosierknopf herauszuschieben und somit zum Beispiel für eine Dosiereinstellung frei zu geben.

Vorzugsweise kann das Dosierelement so mit der Injektionsvorrichtung gekoppelt sein, dass es in einem in die Injektionsvorrichtung eingeschobenen Zustand nicht drehbar ist und zum Beispiel erst nach dem Ausschieben zur Einstellung einer Dosis gedreht werden kann, wobei bezüglich der konkreten Ausführungsform zur Dosiseinstellung auf spätere Ausführungen verwiesen wird. Das Dosierelement kann so ausgebildet sein, dass es nach dem Einstellen der abzugebenden Dosis in einer zum Beispiel vorgegebenen definierten Ausschubposition aus der Injektionsvorrichtung, in welcher es zur Einstellung der Dosis drehbar ist, weiter aus der Injektionsvorrichtung herausgezogen werden kann, um die Injektionsvorrichtung zu laden, wobei das Dosierelement dann zum Beispiel während des weiteren Ausziehvorganges drehgesichert oder auch drehbar sein kann. Ebenso ist es auch möglich, dass die Einstellung der Dosis erst nach dem Laden oder Herausziehen des Dosierelements aus der Injektionsvorrichtung erfolgt und das Dosierelement erst drehgesichert ist oder wird, wenn es zur Abgabe der Substanz wieder in die Injektionsvorrichtung eingeschoben wird.

Das Einstellelement kann im Ausgangszustand oder im eingeschobenen Zustand zum Beispiel durch Haftreibung oder eine Verrastung in der Injektionsvorrichtung oder einem Gehäuse der Injektionsvorrichtung gehalten werden, wobei die Haftreibungskraft oder die Verrastung durch den Einschub der Substanzaufnahmevorrichtung, wie zum Beispiel des Ampullenhalters, überwunden werden kann, um das Einstellelement aus der Injektionsvorrichtung herauszuschieben. Zum Beispiel kann eine proximale Seite des Substanzaufnahmeelements oder ein daran vorgesehenes Einstellelement-Ausschubelement mit dem Einstellelement oder Dosierelement in Berührung kommen oder über ein oder mehrere weitere z.B. durch eine lösbare Verrastung oder Halterung relativ zur Injektionsvorrichtung gesicherte Elemente, wie z.B. eine Führungshülse, so mit dem Dosierelement verbunden oder gekoppelt sein oder werden, dass eine Einschub- oder Eindrehbewegung der Substanzaufnahmevorrichtung zu einem Herausschieben des Einstell- oder Dosierelementes führt. Ebenso kann ein Rastelement oder ein anderes Feststellelement vorgesehen sein, welches das Dosierelement in dem in die Injektionsvorrichtung eingeschobenen Zustand hält und von dem Substanzaufnahmeelement unmittelbar oder von einem damit gekoppelten Element gelöst werden kann, um einen Ausschub des Dosierelementes zu ermöglichen.

Vorzugsweise ist in der Injektionsvorrichtung eine Kolbenstange oder Zahnstange so vorgesehen, dass ein in der Substanzaufnahmevorrichtung oder in einer Ampulle vorgesehener proximaler Stopfen oder Verdrängungskörper während des Einschub- oder Eindrehvorganges der Substanzaufnahmevorrichtung oder der Ampulle in die Injektionsvorrichtung relativ zum Substanzaufnahmebereich verschoben, also zum Beispiel in die Ampulle eingeschoben wird, wodurch zum Beispiel bei einer Zweikammerampulle eine Abmischung der in der Zweikammerampulle enthaltenen Komponenten initiiert wird und bei ausreichendem Einschieben der Kolben- oder Zahnstange abgeschlossen werden kann, so dass zum Beispiel eine Zweikammerampulle zur Vorbereitung der Substanzabgabe abgemischt werden kann.

Bevorzugt ist die Kopplung zwischen Substanzaufnahmeelement und Einstell- oder Dosierelement so ausgebildet, dass das Dosierelement erst nach dem vollständigen oder fast vollständigen Einbringen oder Einschieben des Substanzaufnahmeelementes, zum Beispiel zur vollständigen oder fast vollständigen Abmischung der zum Beispiel in einer Zweikammerampulle enthaltenen Substanzen, aus der Injektionsvorrichtung ausgeschoben wird. Zum Beispiel kann eine Kopplung des Dosierelements mit dem Substanzaufnahmeelement erst im letzten Teilstück des Einschubweges, zum Beispiel auf die letzten 6 mm, stattfinden und das Dosierelement erst nach der Kopplung, zum Beispiel nach dem Anschlag des proximalen Endes des Substanzaufnahmeelementes am Dosierelement, um den verbleibenden Einschubweg von zum Beispiel 6 mm aus der Injektionsvorrichtung herausgeschoben werden, so dass das Dosierelement von einem Benutzer gegriffen und zum Beispiel zur Dosiereinstellung gedreht oder zum Aufziehen des Pens weiter herausgezogen werden kann.

Vorteilhaft ist auf dem Dosierelement, also zum Beispiel auf der Umfangsseite des Dosierknopfes oder einer Dosierknopfhülse, eine Markierung vorgesehen, welche als Hilfe zur Einstellung einer Dosis aufgezeichnet sein kann und zum Beispiel Dosiseinheiten angeben kann. Diese Dosisanzeige kann so auf dem Dosierelement aufgezeichnet oder aufgebracht sein, dass sie erst nach dem Ausschieben des Dosierelementes sichtbar ist, indem zum Beispiel das Dosierelement soweit ausgeschoben wird, dass die Dosisanzeige für einen Benutzer erkennbar wird, oder dass die auf dem Dosierelement aufgezeichnete Anzeige in den Bereich eines zum Beispiel im Gehäuse der Injektionsvorrichtung vorgesehenen Sichtfensters verschoben und somit erkennbar wird.

Bei einem erfindungsgemäßen Verfahren zum Vorbereiten einer Injektionsvorrichtung zur dosierten Abgabe einer Substanz wird ein in die Injektionsvorrichtung eingeschobenes und von einem Benutzer zum Beispiel nicht oder nur schwer greifbares Dosierelement erst während oder nach dem Einschieben eines Substanzaufnahmeelements, wie zum Beispiel eines Ampullenhalters oder einer Ampulle, in die Injektionsvorrichtung aus dieser ausgeschoben, so dass das Einstell- oder Dosierelement zum Beispiel erst dann von einem Benutzer greifbar ist und betätigbar ist.

Gemäß einem weiteren Aspekt bezieht sich die Erfindung auf eine Injektionsvorrichtung mit einem Gehäuse und einem Substanzabgabeelement, welches relativ zu dem Gehäuse bewegbar ist, um durch eine Bewegung des Abgabeelementes, zum Beispiel einen Einschub des Abgabeelementes in die Injektionsvorrichtung, eine Verdrängung einer in der Injektionsvorrichtung enthaltenen Substanz aus der Injektionsvorrichtung zur Abgabe zu bewirken. Erfindungsgemäß ist mindestens ein Feststellelement in der Injektionsvorrichtung vorgesehen, welches nach einer einmal mittels des Abgabeelementes erfolgten Abgabe einer Substanz aus der Injektionsvorrichtung verhindert, dass mittels des Abgabeelementes eine weitere Dosisabgabe durchgeführt werden kann. Das Abgabeelement kann mit einem Dosier- oder Einstellelement gekoppelt oder sogar mit diesem identisch sein, so dass zum Beispiel eine Dosis durch eine Drehung an dem Abgabeelement eingestellt, durch Herausziehen des Abgabeelementes die Injektionsvorrichtung aufgezogen und durch Einschub des Abgabeelementes die tatsächliche Abgabe der Substanz bewirkt werden kann. Insbesondere kann das Abgabeelement auch ein Dreh- oder Dosierknopf der Injektionsvorrichtung sein. Ebenso kann das Abgabeelement auch ein Gehäuseteil der Injektionsvorrichtung sein, der relativ zu einem anderen Element oder Gehäuseteil der Injektionsvorrichtung bewegbar ist.

Erfindungsgemäß ist an dem Abgabeelement ein Feststell- oder Rastelement, also zum Beispiel eine Rastnocke und/oder eine Nut vorgesehen, welche mit einem entsprechenden Gegenelement, also einer korrespondierenden Nut oder Rastnocke der Injektionsvorrichtung oder einer Ampulle, eine nicht oder nur sehr schwer lösbare Verbindung, insbesondere eine Rastverbindung während oder nach einem Abgabevorgang herstellen kann, wenn eine einmal eingestellte Dosis aus der Injektionsvorrichtung abgegeben wird. Das Feststell- oder Rastelement kann zum Beispiel mit einer Kolben- oder Zahnstange der Injektionsvorrichtung zusammenwirken und zum Beispiel kann das Gegenelement als ein in das Abgabeelement hineinragendes und zum Beispiel an einem flexiblen Arm vorgesehenes Rastelement, wie zum Beispiel als Rastnocke, ausgebildet sein, mit welchem eine formschlüssige Verbindung zwischen dem Abgabeelement und der Zahn- oder Kolbenstange nach Abgabe der Substanz hergestellt werden kann. Das Abgabeelement ist dann nicht mehr relativ zur Zahn- oder Kolbenstange bewegbar. Ebenso kann das Abgabeelement relativ zur Injektionsvorrichtung oder einem Gehäuse davon zum Beispiel durch eine Verrastung während oder nach einem Abgabevorgang festgestellt werden, um so einen weiteren Abgabevorgang zu verhindern. Ebenso ist es möglich lediglich eine Bewegung des Abgabeelementes in eine bestimmte Richtung, also zum Beispiel eine axiale Bewegung und/oder eine Drehbewegung, zu sperren oder zu blockieren, um eine durch die gesperrte(n) Bewegungsrichtung(en) nicht mehr mögliche weitere Substanzabgabe aus der Injektionsvorrichtung zu verhindern.

Bei einem erfindungsgemäßen Verfahren zur Abgabe einer Substanz aus der Injektionsvorrichtung oder zur Blockierung der Injektionsvorrichtung wird ein die Substanzabgabe auslösendes oder bewirkendes Abgabeelement vor, während oder nach der Abgabe der eingestellten Dosis der abzugebenden Substanz festgestellte oder arretiert, so dass das Abgabeelement nicht mehr in eine Richtung bewegt werden kann, durch welche eine weitere Abgabe einer Substanz aus der Injektionsvorrichtung ausgelöst werden kann.

Somit kann eine einmal durch einen Benutzer eingestellte oder voreingestellte Menge oder Dosis einer abzugebenden Substanz unter Verwendung der erfindungsgemäßen Injektionsvorrichtung und des erfindungsgemäßen Verfahrens bevorzugt nur ein einziges Mal aus der Injektionsvorrichtung abgegeben werden, wobei weitere Substanzabgaben blockiert werden können, selbst wenn noch eine Restmenge einer abzugebenden Substanz in der Injektionsvorrichtung verbleibt. Dies ist insbesondere bei abzumischenden Substanzen in Zweikammerampullen erforderlich, welche sich zum Beispiel zersetzen oder qualitativ verschlechtern können, so dass eine in größerem Zeitabstand nach dem Abmischen der Substanz durchgeführte erneute Substanzabgabe an einen Patienten nicht ratsam und möglicherweise sogar gesundheitsgefährdend sein kann.

Die Erfindung wird nachfolgend anhand bevorzugter Ausführungsbeispiele beschrieben. Es zeigen:
- Figuren 1A bis 1E: den Ablauf einer Dosiseinstellung und Substanzabgabe bei einer Ausführungsform einer erfindungsgemäßen Injektionsvorrichtung jeweils in Draufsicht und im Querschnitt;
- Figuren 2A und 2B: eine Querschnittsansicht einer Injektionsvorrichtung vor und nach dem Ausschieben des Dosierknopfes;
- Figuren 3A und 3B: eine erste Ausführungsform einer Dosier- und Ausschüttmechanik im Querschnitt;
- Figuren 4A und 4B: eine zweite Ausführungsform einer Dosier- und Ausschüttmechanik im Querschnitt;
- Figur 5A: eine Querschnittsansicht eines erfindungsgemäßen Dosierungsknopfes;
- Figur 5B: die Innenseite des in Figur 5A gezeigten Dosierknopfes in abgerollter Darstellung;
- Figuren 6A bis 6C: das Zusammenwirken der erfindungsgemäßen Ausschüttmechanik mit einer Zahnstange gemäß einer Ausführungsform; und
- Figur 6D: die Zahnstange und Ausschüttmechanik in Explosionsdarstellung.

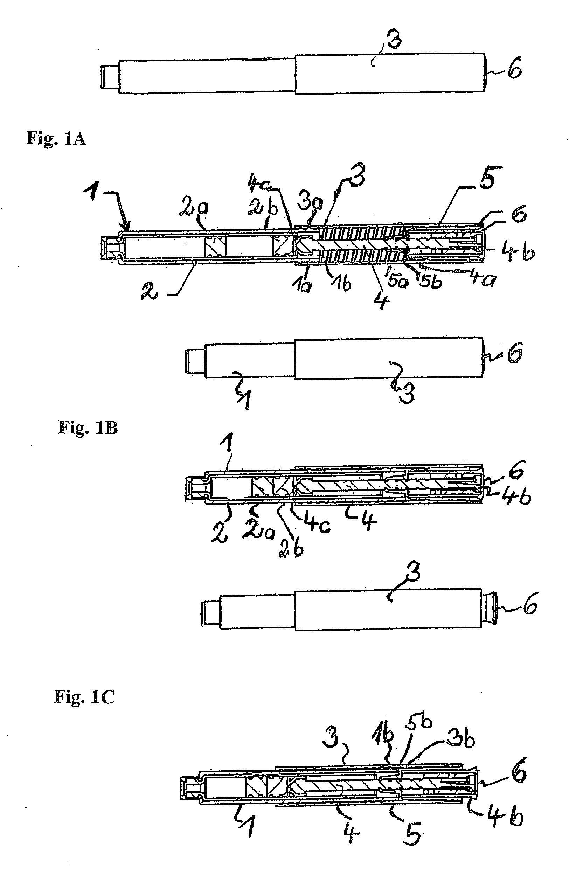

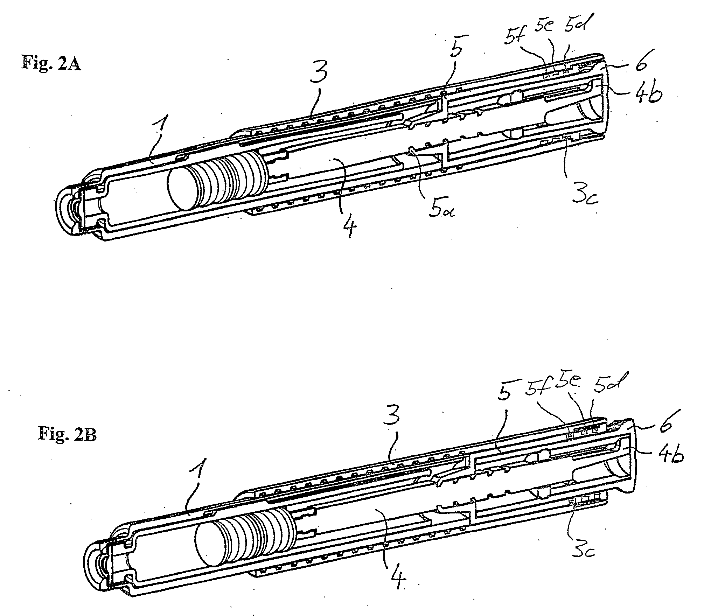

Figur 1A zeigt in Draufsicht und im Querschnitt eine Ausführungsform einer erfindungsgemäßen Injektionsvorrichtung mit einem Ampullenhalter 1 als Substanzaufnahme, in welchen eine Zweikammerampulle 2, in der verschiebbar zwei Stopfen 2a und 2b angeordnet sind, eingelegt werden kann. Der Ampullenhalter 1 ist relativ zum Gehäuse der Injektionsvorrichtung, die in der gezeigten Ausführungsform durch die Gewindehülse 3 gebildet wird, verschiebbar und kann in das Innengewinde 3a der Gewindehülse 3 mittels des Eingriffes des am proximalen Ende des Ampullenhalters 1 vorgesehenen Außengewindes 1a eingeschraubt werden. Eine Führungshülse 5 ist durch den Eingriff des auf der Innenseite der Gewindehülse 3 vorgesehenen Ringsteges 3c in die auf der Aussenseite der Führungshülse 5 umlaufende Rille 5d (siehe Figur 2A) mit der Gewindehülse 3 verbunden und weist an ihrem distalen Ende radial nach innen vorgespannte Rastelemente 5a auf, welche in korrespondierende Nuten oder eine Zahnung 4a einer z.B. durch axial verlaufende Rillen drehgesicherten Zahnstange 4 eingreifen und diese gegen ein Verschieben in proximaler Richtung halten. Jedoch kann die Zahnstange 4 in distaler Richtung relativ zur Führungshülse 5 bewegt werden, wobei die an elastischen Armen vorgesehenen Rastelemente 5a aus den Nuten oder der Zahnung 4a der Zahnstange herausgleiten und in eine nachfolgende Nut oder Zahnung 4a einschnappen. In der gezeigten Ausführungsform ist am proximalen Ende ein als Abgabe- und Dosierelement 6 ausgebildeter Dosierknopf vorgesehen, welcher in das Gehäuse oder in die Gewindehülse 3 so weit eingeschoben ist, dass er praktisch nicht von einem Benutzer gefasst werden kann. In der in Figur 1A gezeigten Grundstellung der Injektionsvorrichtung, welche auch als Auslieferungsposition bezeichnet werden kann, lässt sich nur der Ampullenhalter 1 bewegen, also in die Gewindehülse 3 einschrauben, um die in der Zweikammerampulle 2 befindlichen Substanzen abzumischen. Jedoch ist noch kein Einstellvorgang mittels des Dosierknopfes 6 möglich.

Figur 1B zeigt die in Figur 1A gezeigte Injektionsvorrichtung in Mischposition nachdem der Ampullenhalter 1 in die Gewindehülse 3 eingedreht wurde. Durch das Verschieben der von dem Ampullenhalter 1 gehaltenen Ampulle 2 in proximale Richtung wird der auf der Vorderseite der Zahnstange 4 aufgesetzte Aufsatz 4c in die Ampulle 2 hineinbewegt, wodurch der anliegende Stopfen 2b in die Ampulle 2 hinein geschoben wird, um die Ampulle 2 auf bekannte Art abzumischen. Gleichzeitig kann auch geprimt werden.

Wie in Figur 1C gezeigt, kann der Ampullenhalter 1 so weit in die Gewindehülse 3 der Injektionsvorrichtung eingeschraubt werden, bis eine proximale Anschlagfläche 1b des Ampullenhalters 1 an der radial äußeren distalen oder Vorderseite der Führungshülse 5 ansteht, wodurch bei weiterem Einschrauben des Ampullenhalters 1 die Führungshülse 5 zusammen mit dem Ampullenhalter 1 in proximale Richtung relativ zur Gewindehülse 3 bewegt wird, wobei die Rastverbindung 3c, 5d gelöst wird, bis ein auf der Außenseite der Führungshülse 5 vorgesehenes Anschlagelement 5b gegen ein Gegenanschlagelement 3b auf der Innenseite der Gewindehülse 3 bewegt wird, wodurch die Eindrehbewegung des Ampullenhalters 1 begrenzt wird. Durch die Verschiebung der Führungshülse 5 in proximale Richtung wird der in der Führungshülse 5 gelagerte Dosierknopf 6 in proximaler Richtung aus der Injektionsvorrichtung herausgeschoben, so dass der Dosierknopf 6 verwendet werden kann, um eine aus der Injektionsvorrichtung abzugebende Dosis einzustellen. Dabei kommt der Ringsteg 3c mit den umlaufenden Rillen 5e (Priming-Position) und 5f (Endposition) der durch den an der Führungshülse 5 anliegenden Ampullenhalter 1 vorbeigeschobenen Führungshülse 5 in Eingriff.

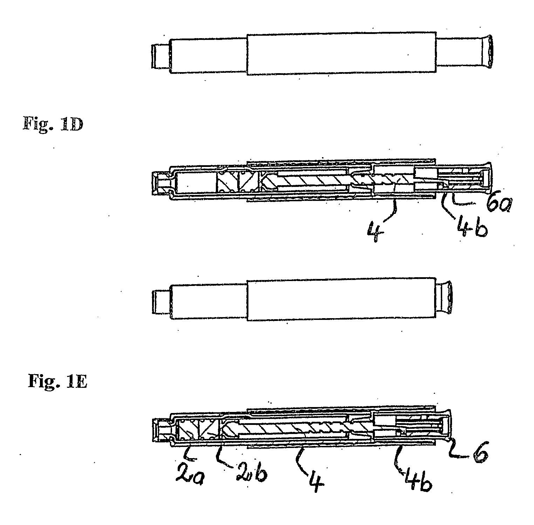

Figur 1D zeigt die geladene Injektionsvorrichtung, wobei der Dosierknopf 6 aus der Injektionsvorrichtung herausgezogen wurde, bis ein an der Zahnstange 4 vorgesehenes in proximale Richtung ragendes Rastelement 4b in eine an der Innenseite des Dosierknopfes 6 vorgesehene Rastöffnung 6a einrastet, so dass durch einen Druck auf den Dosierknopf 6 die so mit diesem verrastete Zahnstange 4 in distale Richtung der Injektionsvorrichtung geschoben werden kann, wie in Figur 1E gezeigt, um hierdurch die in der Ampulle 2 befindlichen Stopfen 2a und 2b in distale Richtung zu verschieben und das bereits abgemischte Medikament aus der Ampulle 2 abzugeben. Ist der Dosierknopf 6 wieder in den Pen bzw. in die Gewindehülse 3 eingeschoben, kann dieser, wie nachfolgend erläutert, nicht mehr bewegt und insbesondere nicht mehr herausgezogen werden. Somit kann sichergestellt werden, dass die erfindungsgemäße Injektionsvorrichtung nur für einen einzigen Ausschüttvorgang verwendet werden kann.

Figur 2A zeigt in perspektivischer Querschnittsansicht den wie in der Ausgangsstellung gezeigten eingeschobenen Dosierknopf 6, welcher durch Eindrehen oder Einschieben des Ampullenhalters 1 mittels der Führungshülse 5 aus der Injektionsvorrichtung herausgeschoben und somit für einen Benutzer freigestellt werden kann; siehe Figur 2B.

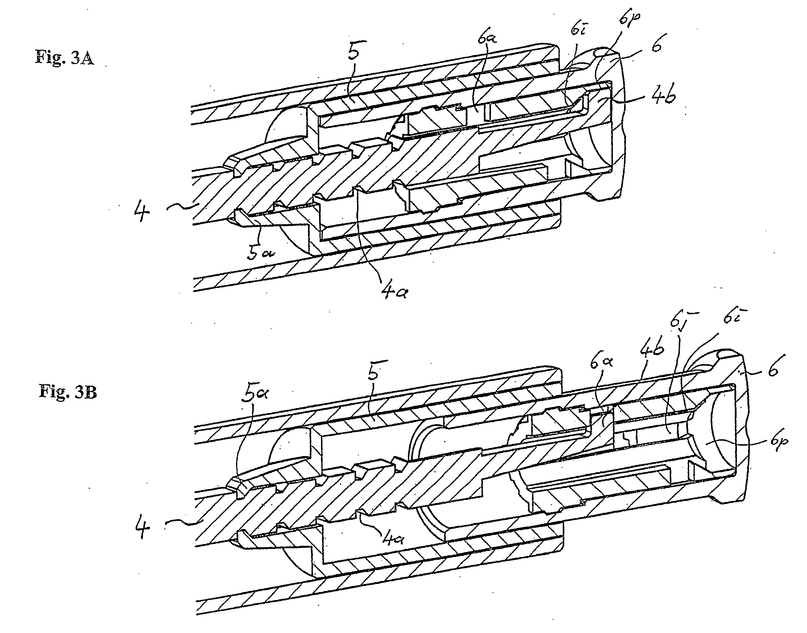

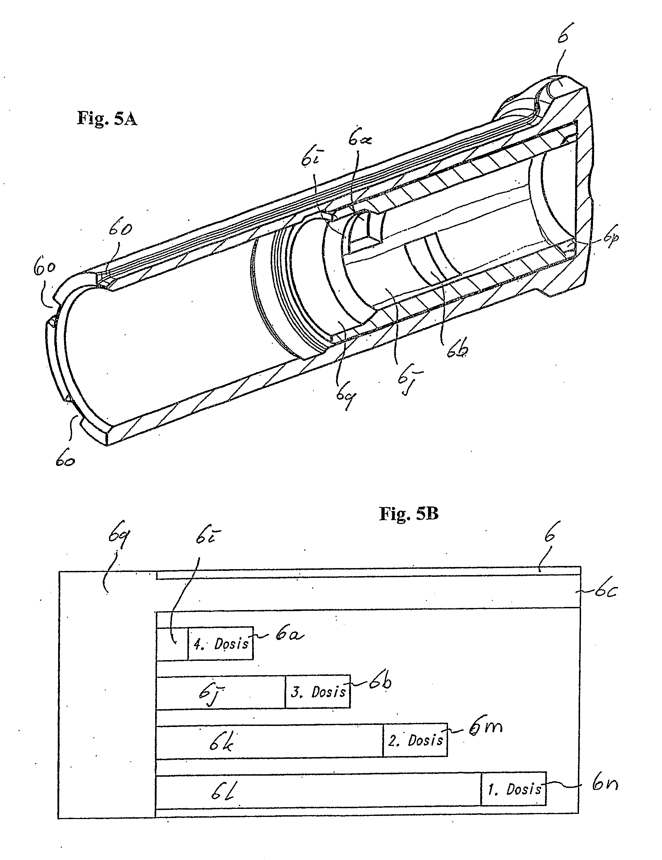

Figur 3A zeigt in perspektivischer Querschnittsansicht eine erste Ausführungsform einer erfindungsgemäßen Einstell- und Ausschubmechanik, wobei in der gezeigten Grundstellung der Dosierknopf 6 zur Dosiswahl frei drehbar ist und der radial nach außen vorgespannte Schnapper oder das Rastelement 4b der Gewindestange 4 in der an der proximalen inneren Seite des Dosierknopfes 6 umlaufenden Nut 6p frei umlaufen kann, um so auch den Dosierknopf 6 zu halten. Ist eine Dosis zum Beispiel anhand einer auf der äußeren Umfangsseite des Dosierknopfes 6 angebrachten Markierung eingestellt, so kann der Dosierknopf 6 aus der Injektionsvorrichtung herausgezogen werden, wie in Figur 3B gezeigt, bis das radial nach außen vorgespannte Rastelement 4b der Gewindestange 4 durch eine Nut 6i an der Innenseite des Dosierknopfes 6 geführt in eine von zum Beispiel mehreren im Inneren des Dosierknopfes 6 vorgesehene Rastöffnungen 6a oder 6b (mit Führungsnuten 6i oder 6j; siehe Figur 5B) einrastet und die eingestellte Dosis durch Einschieben des Dosierknopfes 6 ausgeschüttet werden kann, welcher über die formschlüssige Verbindung 6a, 4b die Gewindestange 4 in distale Richtung mitnimmt und relativ zur Führungshülse 5 verschiebt. Greift das Rastelement 4b in die Nut 6i des Dosierknopfes 6 ein, wie in Figur 3B gezeigt, so ist der Dosierknöpf nicht mehr frei drehbar, aber axial in der Nut 6i verschiebbar, bis er in die Rastöffnung 6a einrastet. Nach dem Einschieben in den Pen kann der Dosierknopf 6 aufgrund dieser Verbindung 4b, 6a, sowie aufgrund der Rastverbindung 4a, 5a nicht mehr bewegt werden. Die Rastverbindung 3c, 5f zwischen Gewindehülse 3 und Führungshülse 5 ist stärker als die Rastverbindung 4a, 5a zwischen Führungshülse 5 und Zahnstange 4, so dass ein Druck auf den Dosierknopf 6 zu einer axialen Vorschubbewegung der Zahnstange 4 relativ zur Führungshülse 5 führt.

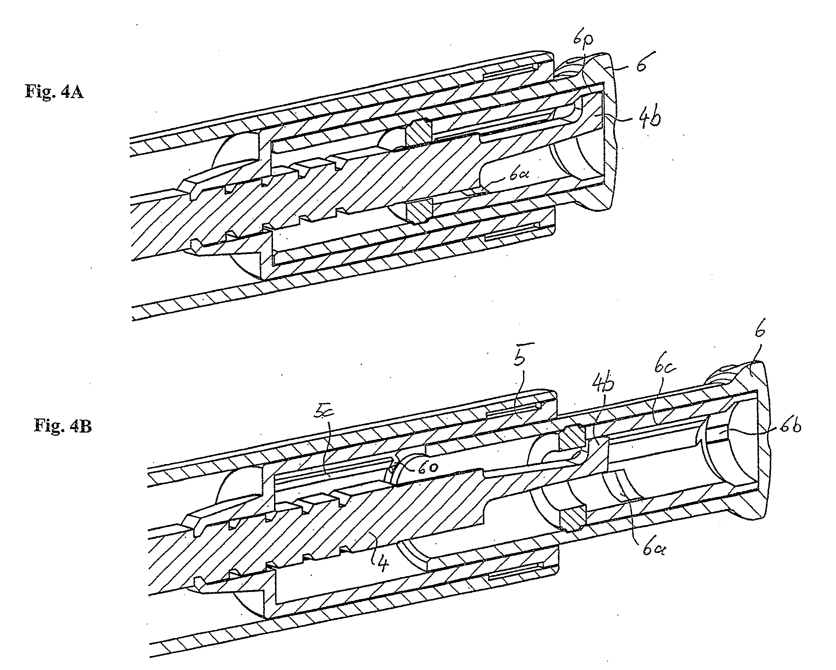

Figur 4A zeigt zusammen mit Figur 4B eine zweite Ausführungsform einer erfindungsgemäßen Einstell- und Ausschüttmechanik, wobei in der in Figur 4A gezeigten Grundstellung der von dem Rastelement 4b durch Eingriff in die Nut 6p lösbar gehaltene Dosierknopf 6 aufgrund des Eingriffs eines oder mehrerer Führungsstäbe 5c (siehe Figur 4B) an der Innenseite der Führungshülse 5 in korrespondierende Nuten oder Rillen 6o an der Außenseite des Dosierknopfes 6 radial nicht drehbar ist.

Wird der Dosierknopf 6 aus der Injektionsvorrichtung herausgezogen, wie in Figur 4B gezeigt, so wird die Drehsicherung des Dosierknopfes 6 durch Herausschieben der Rillen 6o aus den Führungsstäben gelöst, das Rastelement 4b läuft durch die Leernut 6c des Dosierknopfes 6 in die auf der Innenseite des Dosierknopfes 6 umlaufende Einstellnut 6q und der Dosierknopf 6 kann zur Dosiswahl frei gedreht werden. Die Dosis wird durch auf der Innenseite des Dosierknopfes 6 angeordnete in Umfangsrichtung und axial zueinander versetzte Raststellen, Vertiefungen oder Öffnungen 6a, 6b, 6m, 6n mit zugehörigen Führungsrillen 6i bis 61 (siehe Figur 5B) mit unterschiedlicher axialer Länge festgelegt. Beim Einschieben des Dosierknopfes 6 fährt das Rastelement 4b entlang der durch die Drehposition des Dosierknopfes 6 festgelegte Führungsrille 6i, 6j, 6k oder 61, bis es an der zugeordneten Raststelle 6a, 6b, 6m oder 6n einrastet. Wird der Dosierknopf 6 weiter eingeschoben, so nimmt dieser die über die Rastverbindung des Rastelementes 4b gekoppelte Zahnstange 4 mit. Ist der Dosierknopf 6 vollständig eingeschoben, so kann der Dosierknopf 6 aufgrund der Drehsicherung 5c, 6o und der Kopplung mit der in proximale Richtung nicht mehr herausziehbaren Zahnstange 4 nicht mehr bewegt werden, so dass die Injektionsvorrichtung gesperrt ist.

Figur 5A zeigt eine Ausführungsform eines für beide oben beschriebenen Varianten verwendbaren Dosierknopfes 6 in perspektivischer Schnittansicht, wobei auf der Innenseite in Umfangsrichtung und axialer Richtung zueinander versetzt verschiedene Rastöffnungen 6a, 6b vorgesehen sind, so dass durch eine Drehstellung des Dosierknopfes 6 eine jeweils in verschiedener axialer Richtung liegende Einrastmöglichkeit in einer der Rastöffnungen 6a, 6b für das Rastelement 4b vorgegeben werden kann. Die Zahnstange 4 wird somit bei der unter Verweis auf Figur 3 beschriebenen ersten Ausführungsform auch beim vollständigen Herausziehen des Knopfes 6 nur so weit herausgezogen, wie durch die Drehstellung und die Öffnungen 6a, 6b, 6m, 6n des Knopfes 6 vorgegeben. Demgegenüber wird bei der zweiten unter Verweis auf Figur 4 beschriebenen Ausführungsform die Zahnstange bis zum Verrasten nur so weit eingeschoben, wie durch die Drehstellung und die Öffnungen 6a, 6b, 6m, 6n des Knopfes 6 vorgegeben.

Figur 5B zeigt in abgerollter Darstellung das Innenprofil des Dosierknopfes 6, wobei in Abhängigkeit von der Drehstellung des Dosierknopfes 6 durch die unterschiedlich langen Nuten 6i bis 61 zum Einschub des Rastelementes 4b unterschiedliche Schubwege der Zahnstange 4 vorgegeben werden können und somit unterschiedliche Dosen ausgeschüttet werden können. In Figur 5B ist mit "1. Dosis" die kleinste und mit "4. Dosis" die grösste bei der zweiten Ausführungsform auszuschüttende Dosis vorgegeben.

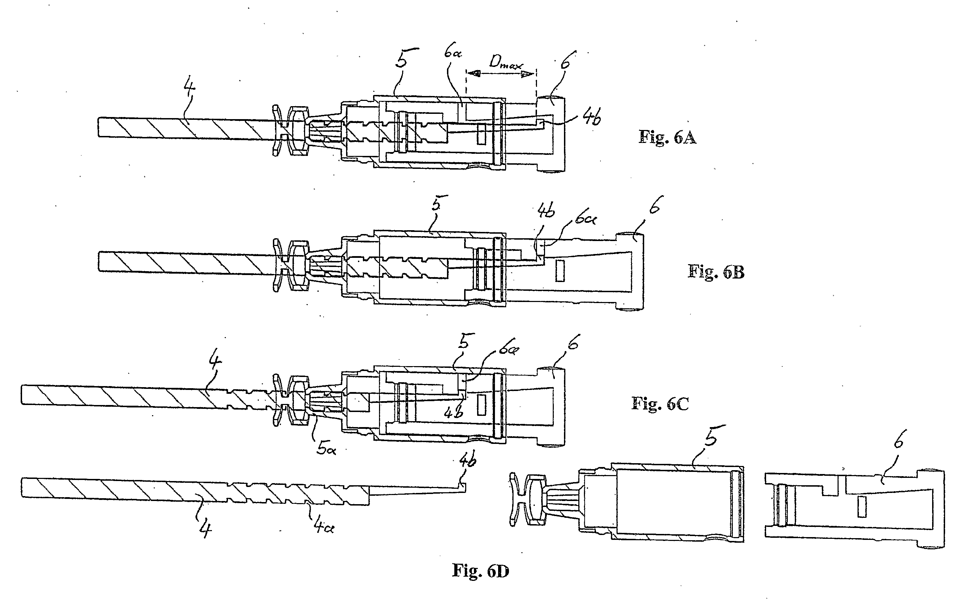

Figur 6A zeigt das Zusammenwirken zwischen Einstell- und Dosiermechanik mit der Zahnstange 4 gemäß der in Figur 3 gezeigten ersten Ausführungsform, wobei in der in Figur 6A gezeigten Querschnittsansicht die maximale Hub- oder Ausschüttbewegung mit der gezeigten Mechanik als Abstand zwischen dem Rastelement 4b der Zahnstange 4 und der Rastöffnung 6a des Dosierknopfes 6 mit Dₘₐₓ bezeichnet ist.

Wird der Dosierknopf 6 herausgezogen, wie in Figur 6B gezeigt, so rastet das Rastelement 4b in der Rastöffnung 6a ein und der Dosierknopf 6 ist nach dem Einrasten in der herausgezogenen Stellung nicht mehr drehbar und kann zum Ausschütten der Dosis eingeschoben werden, wie in Figur 6C gezeigt. In der eingeschobenen Stellung verhindern die Rastnasen 5a der Führungshülse 5 ein erneutes Herausziehen der Zahnstange 4 und damit des mit der Zahnstange 4 gekoppelten Dosierknopfes 6, so dass der Dosierknopf 6 in der in Figur 6C gezeigten Stellung festgestellt ist und die Injektionsvorrichtung nicht mehr betätigt werden kann.

Statt einer Verrastung oder Rastöffnung, wie z.B. unter Bezugnahme auf die Figuren 3 bis 6 beschrieben, können zur Dosiseinstellung oder Dosiswahl natürlich auch ein oder mehrere Anschläge, Gegenanschläge, Stufen, vorstehende Elemente, Stege oder Nocken z.B. an dem Dosierknopf 6 vorgesehen sein. Ebenso können die Nuten 6i bis 61 ohne anschliessende Öffnungen als Sacknuten oder Sackratten ausgebildet sein.

## Patentansprüche

1. Injektionsvorrichtung mit einer relativ zur Injektionsvorrichtung oder relativ zu einer Halterung (5) der Injektionsvorrichtung in nur die distale Richtung bewegbaren Vorschubstange (4) mit mindestens einem Halte- oder Rastbereich, insbesondere einer Zahnung (4a), zum lösbaren Halten der Vorschubstange (4) an oder in der Injektionsvorrichtung oder der Halterung (5), **gekennzeichnet durch** ein an der Vorschubstange (4) vorgesehenes Koppelelement (4b) zum Positionieren der Vorschubstange (4) relativ zu einem Dosierelement (6), insbesondere zum Festlegen der axialen Lage der Vorschubstange (4) relativ zu dem Dosierelement (6), um eine nicht oder nur sehr schwer lösbare Verbindung zwischen der Vorschubstange (4) und dem Dosierelement (6) während oder nach einem Abgabevorgang herzustellen, um eine weitere Dosisabgabe zu verhindern, wobei an dem Dosierelement (6) mehrere unterschiedliche Gegenanschläge oder Vertiefungen (6a, 6b, 6m, 6n) vorgesehen sind, die einen oder mehrere Gegenanschläge oder Eingriffspositionen für das Koppelelement (4b) der Vorschubstange bilden.

2. Injektionsvorrichtung nach Anspruch 1, wobei das Koppelelement (4b) ein radial nach aussen vorstehendes Element, ein Rastelement oder ein Anschlagelement ist.

3. Injektionsvorrichtung nach einem der vorhergehenden Ansprüche, wobei das Koppelelement (4b) asymmetrisch aufgebaut ist.

4. Injektionsvorrichtung nach einem der vorhergehenden Ansprüche, wobei die Vorschubstange (4) unmittelbar oder mittelbar auf einen Verdrängungskörper, insbesondere einen Stopfen (2b) einer Ampulle (2) drücken kann.

5. Injektionsvorrichtung nach einem der vorhergehenden Ansprüche, wobei das Dosierelement (6) ein Treppenglied mit in Umfangsrichtung versetzten Vertiefungen oder Löchern unterschiedlicher axialer Länge ist.

6. Injektionsvorrichtung nach einem der vorhergehenden Ansprüche, wobei das Dosierelement (6) eine Verzahnung aufweist, in die das Koppelelement zur Einstellung der Dosis eingreift.

7. Injektionsvorrichtung nach einem der vorhergehenden Ansprüche, wobei das Koppelelement (4b) in Relation zu dem Halte- oder Rastbereich, insbesondere der Zahnung (4a), in proximale Richtung versetzt angeordnet ist.

## Claims

1. Injection device with an advancing rod (4), moveable relative to the injection device or relative to a holder (5) of the injection device only in the distal direction, with at least one holding or arresting area, in particular a toothing (4a), for the disconnectable holding of the advancing rod (4) on or in the injection device or the holder (5), **characterised by** a coupling element (4b) envisaged on the advancing rod (4) for positioning the advancing rod (4) relative to a dosing element (6), in particular for fixing the axial position of the advancing rod (4) relative to the dosing element (6) for producing a disconnectable connection, or one that is difficult to disconnect, between the advancing rod (4) and the dosing element (6) during or after a dispensing process, for preventing further dose dispensation, wherein several different counterstops or recesses (6a, 6b, 6m, 6n) are envisaged on the dosing element (6), which form one or more counterstops or engagement positions for the coupling element (4b) of the advancing rod.

2. Injection device according to claim 1, wherein the coupling element (4b) is a radially outward projecting element, an arresting element or a stop element.

3. Injection device according to one of the preceding claims, wherein the coupling element (4b) is constructed asymmetrically.

4. Injection device according to one of the preceding claims, wherein the advancing rod (4) can press directly or indirectly on a displacement body, in particular a plug (2b) of a vial (2).

5. Injection device according to one of the preceding claims, wherein the dosing element (6) is a step member with recesses or holes of different axial lengths offset in a circumferential direction.

6. Injection device according to one of the preceding claims, wherein the dosing element (6) comprises a toothing that engages the coupling element for adjusting the dose.

7. Injection device according to one of the preceding claims, wherein the coupling element (4b) is position-offset in relation to the holding or arresting area, in particular the toothing (4a) in a proximal direction.

## Revendications

1. Dispositif d'injection avec une tige d'avance (4) mobile par rapport au dispositif d'injection ou par rapport à un support (5) du dispositif d'injection seulement dans la direction distale avec au moins une zone de retenue ou d'encliquetage, en particulier une denture (4a) pour le maintien amovible de la tige d'avance (4) sur ou dans le dispositif d'injection ou le support (5), **caractérisé par** un élément de couplage (4b) prévu sur la tige d'avance (4) pour le positionnement de la tige d'avance (4) par rapport à un élément de dosage (8), en particulier pour la fixation de la position axiale de la tige d'avance (4) par rapport à l'élément de dosage (6) afin d'établir une liaison non amovible ou seulement difficilement entre la tige d'avance (4) et l'élément de dosage (6) pendant ou après un processus d'administration afin d'empêcher une autre administration de dose, dans lequel plusieurs butées antagonistes ou cavités (6a, 6b, 6m, 6n) différentes sont prévues sur l'élément de dosage (6), lesquelles forment une ou plusieurs butées antagonistes ou positions d'engagement pour l'élément de couplage (4b) de la tige d'avance.

2. Dispositif d'injection selon la revendication 1, dans lequel l'élément de couplage (4b) est un élément dépassant radialement vers l'extérieur, un élément d'encliquetage ou un élément de butée.

3. Dispositif d'injection selon l'une quelconque des revendications précédentes, dans lequel l'élément de couplage (4b) est constitué de manière asymétrique.

4. Dispositif d'injection selon l'une quelconque des revendications précédentes, dans lequel la tige d'avance (4) peut presser directement ou indirectement sur un corps de déplacement, en particulier un bouchon (2b) d'une ampoule (2).

5. Dispositif d'injection selon l'une quelconque des revendications précédentes, dans lequel l'élément de dosage (6) est un organe échelonné avec des cavités ou trous décalés dans le sens périphérique de différente longueur axiale.

6. Dispositif d'injection selon l'une quelconque des revendications précédentes, dans lequel l'élément de dosage (6) présente une denture, dans laquelle l'élément de couplage s'engage pour le réglage de la dose.

7. Dispositif d'injection selon l'une quelconque des revendications précédentes, dans lequel l'élément de couplage (4b) est agencé en déport par rapport à la zone de retenue ou d'encliquetage, en particulier à la denture (4a) dans le sens proximal.
